Europäisches Patentamt

European Patent Office

Office européen des brevets

(11) Publication number: **0 486 233 B1**

# EUROPEAN PATENT SPECIFICATION

(45) Date of publication of patent specification: **19.07.95**  (51) Int. Cl.6: **C12N 15/82**, C12N 15/87

(21) Application number: **91310374.3**

(22) Date of filing: **11.11.91**

(54) **Plant transformation method using agrobacterium species.**

(30) Priority: **14.11.90 US 614402**

(43) Date of publication of application:
**20.05.92 Bulletin 92/21**

(45) Publication of the grant of the patent:
**19.07.95 Bulletin 95/29**

(84) Designated Contracting States:
**AT BE CH DE DK ES FR GB GR IT LI LU NL SE**

(56) References cited:

PLANT PHYSIOLOGY.SUPPL. vol. 96, no. 1, 1991, ROCKVILLE, MD, USA. page 95; BIDNEY, D., ET AL.: 'Wounding of tobacco leaves by microprojectile bombardment increases transformation frequency by Agrobacterium tumafaciens'

PLANT MOLECULAR BIOLOGY. vol. 18, no. 2, January 1992, DORDRECHT, THE NETHERLANDS. pages 301 - 311; BIDNEY, D., ET AL.: 'Microprojectile bombardment of plant tissue increases transformation frequency by Agrobacteriumtumefaciens'

(73) Proprietor: **PIONEER HI-BRED INTERNATIONAL, INC.**
**700 Capital Square**
**400 Locust Street**
**Des Moines**
**Iowa 50309 (US)**

(72) Inventor: **Bidney, Dennis**
**8385 Plum Drive**
**Des Moines,**
**Iowa 50323 (US)**

(74) Representative: **Goldin, Douglas Michael et al**
**J.A. KEMP & CO.**
**14, South Square**
**Gray's Inn**
**London WC1R 5LX (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Technical Field

The present invention relates to the use of Agrobacterium species for the transformation of plants.

### Background Art

Much research in plant molecular biology is now directed to the improvement of plant varieties via use of recombinant DNA techniques. Historically, plant breeders used classical genetic techniques to identify, preserve and crossbreed varietal lines having desirable traits. More recently, new plant varieties were induced by chemicals or by radiation treatment to mutate plant cells which were then regenerated using tissue culture techniques. These random and unpredictable approaches have obvious drawbacks. By the use of recombinant DNA technology, specific genes producing specific proteins, such as those imparting insect resistance, can be introduced into a plant to produce a desired variety with a particular trait.

Plants have been transformed using a variety of methods. A common method for transformation of dicotyledonous plants has been the use of disarmed Agrobacterium species, which are relatively benign natural pathogens of dicotyledonous plants. Agrobacteria infect plants and cause a callus of tumor tissue to grow in an undifferentiated way at the site of infection. The tumor inducing agent is the Ti plasmid, which functions by integrating some of its DNA into the genome of host plant cells. This plasmid is an ideal vector for transformation of plants. The portion of the Ti plasmid DNA that is transferred to host cell chromosomes during Agrobacterium infection is referred to as transforming ("T") DNA. See, for example, Watson JD, Tooze J, & Kurtz DT, Recombinant DNA: A Short Course, 169 (W.H. Freeman, 1983).

While early studies with Agrobacterium suggested that dicots were completely insensitive to this pathogen, those conclusions were based on lack of observable tumor formation in inoculated plants. More recently, it has been found that tumor formation in dicots is attributable to overproduction of auxins and cytokinins caused by the Ti plasmid, and therefore this symptom is not always a reliable indicator of transformation. More sensitive and more recent studies have shown production of opaline and nopaline, also attributed to the Ti plasmid, in Agrobacterium-inoculated monocots, and genetically engineered marker genes, such as GUS and NPTII, have been found in progeny of Agrobacterium-transformed corn plants. However, the successful and reliable use of this method still tends to be genotype specific both as to plants and Agrobacterium, as well as culture medium specific. Even under good conditions, the frequency of transformation is relatively low in some species.

In addition, Agrobacteria normally require a wound environment to induce the DNA transfer needed for transformation. For example, leaf punches and stem segments are commonly used because they present a cut and wounded surface to the bacteria that may contain cells capable of regenerating plants. There are times, however, when the intended target is an organized, multilayered tissue, such as a meristem, which is not readily accessible for Agrobacterium infection and transformation and is not easily wounded without damaging its organization and function. Even where leaf punches and stem segments are used, these only present a limited region, such as the perimeter of a leaf punch disk, which has been wounded. It would be desirable to use the entire surface of the disk as a potential transformation site.

Another method for transformation of plants has been bombardment of plant cells with dense microparticles carrying genetic material such as DNA sequences or plasmids. This method is less genotype specific, but frequencies of stable transformation are also low with this method. This is due in part to an absence of natural mechanisms to mediate integration of the introduced genetic material into the plant genome. In contrast, Agrobacterium species actively mediate those transformation events as a part of the natural process of infecting a plant cell. Thus, a continuing need exists for a method of transformation which reduces genotype specificity and enhances reliability, both in monocots and dicots.

### Brief Description of the Drawings

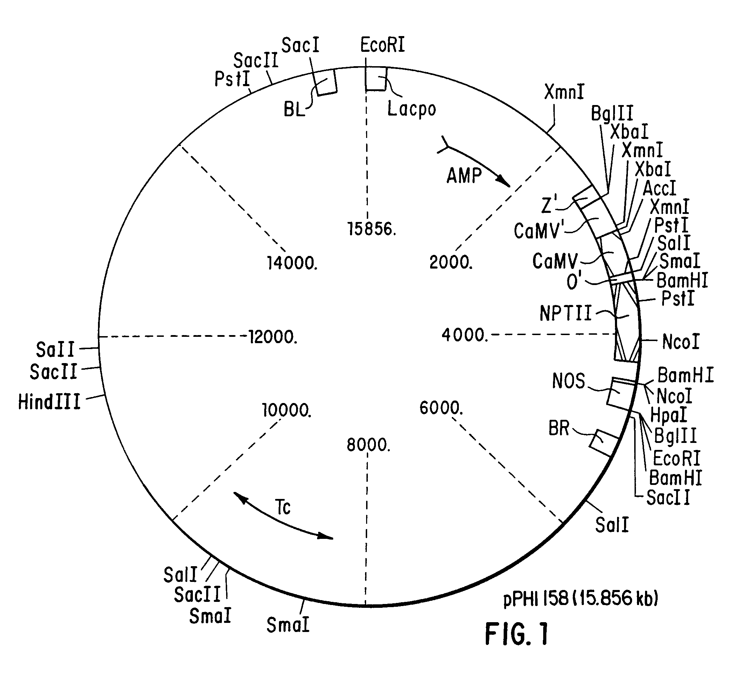

Figures 1 through 4 are plasmid maps of the plasmids pPHI158, pPHI167, pPHI419 and pPHI413, respectively.

### Disclosure of the Invention

This invention provides an improved transformation method in which plant tissues are first perforated with microprojectiles which do not carry genetic material. It is now appreciated that the high velocity impact of dense particles on plant tissues will generate a wide array of microwounds, creating an environment which is particularly conducive to infection by Agrobacteria. In the next step, the tissues are treated with an Agrobacterium species carrying the genetic material of interest. The Agrobacterium is able to transfer genetic material permanently into the genome of target cells at frequencies substantially higher than by conventional Agrobacterium treatment. While excessive wounding of the target tissue is detrimental in conventional particle/ plasmid methods of transformation, such wounding is used to advantage in the present invention. Accordingly, the present invention provides a method for transformation of cells of a plant, comprising the steps of (a) preparing bacteria of an Agrobacterium species, which bacteria have been transformed to include in their T-DNA the genetic material to be inserted into the genome of the plant cells; (b) perforating a tissue from the plant by microprojectile bombardment; and (c) treating the perforated tissue with the transformed Agrobacteria; whereby the Agrobacteria incorporate the T-DNA, including the inserted genetic material, into the genome of the cells.

This method can be used to make permanently, heritably transformed plant cells which can be regenerated to whole, fertile plants. Of course, it will be appreciated that the foregoing method can also be used for transient transformations and assays in plant research.

The transformed plant cells produced by the foregoing method are suitable for regeneration by known techniques to produce whole, fertile plants which include in their nuclear genome the genetic material incorporated by the action of the Agrobacteria. Accordingly, this invention also provides a method of producing whole, fertile, transformed plants, comprising the steps of (a) culturing tissues of the species and genotype to be transformed; (b) preparing bacteria of an Agrobacterium species, which bacteria have been transformed to include in their T-DNA the genetic material to be inserted into the genome of the plant cells; (c) perforating the target tissue by microprojectile bombardment using microprojectiles which do not carry genetic material; (d) treating the perforated tissue with the transformed Agrobacteria, whereby the Agrobacteria incorporate the T-DNA, including the inserted genetic material into the genome of the cells to produce transformed cells; and (e) regenerating the transformed cells to produce whole plants.

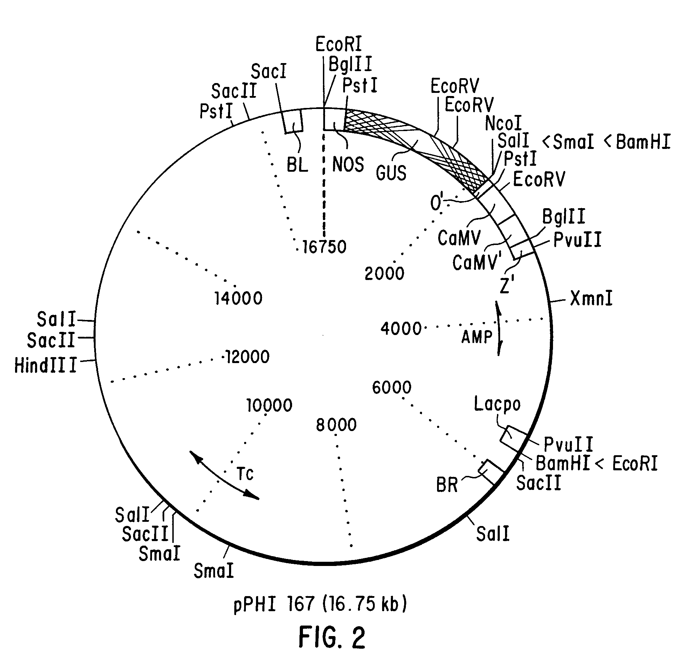

In many instances it will be desirable to regenerate plants from cultures which consist entirely or essentially of transformed cells, so that plants which are not chimeric can be obtained. This can be accomplished by growing the bombarded and Agrobacterium-treated tissue prior to regeneration in a selection medium in which only transformed cells are viable. This can be done by including a selectable marker gene such as kanamycin or Basta resistance in the plasmid to be inserted in the cells, as illustrated in Figure 2. When the treated cells are grown in a medium containing the antibiotic or herbicide, the chemical will destroy non-transformed cells, and the surviving cultures will consist entirely of transformants, which can then be regenerated to produce plants which are not chimeric.

While not intending to be limited by theory, normal microparticle bombardment schemes require that individual or very small groups of particles enter the target cells in such a manner and location that the cells remain competent for division. In contrast, it is believed that Agrobacterium transformation occurs when the bacteria bind to the surface of a target cell. It is only the bacterial T-DNA that is "injected" into the cell, once the bacteria are induced by the wound environment to activate their virulence and transfer functions. Thus it will be appreciated that the objective of bombardment in the practice of this invention is to induce cell wounding and death to a certain extent, rather than to minimize wounding as is desirable with the conventional practice of bombarding with DNA-loaded particles. Once an area is damaged and releases the set of cell metabolites and wound exudates which Agrobacteria recognize, the remaining intact cells in the region of the wound are the transformation targets, rather than the cells which have been hit by particles. Accordingly, in the practice of this invention the particles need not and preferably do not carry genetic or other biological material.

## Plants and Plant Cells

This method can be employed with any desired agronomic or horticultural species, including both monocots and dicots. As evidenced by the results achieved in sunflower, the higher transformation frequencies obtained with this invention can overcome in part the low frequencies of transformation associated with many difficult to tranform genotypes and species. Preferably, the monocot species will be selected from maize, sorghum, triticale, barley, oats, rye, wheat, onion and rice, and the dicot species will be selected from soybean and other beans; alfalfa; tobacco, brassicas such as rapeseed, broccoli and cauliflower; sunflower; cucurbits such as melons, cucumbers and squashes; and solanaceae such as potatoes, peppers and tomatoes. Tissues from flowers, including orchid, rose, carnation, petunia, zinnia, aster, lily, marigold, impatiens, African and common violet and pansy, anthurium, gladiolus, hyacinth, geranium, lavender, peony, tulip, poppy, chrysanthemum, daffodil, and begonia varieties, as well as other ornamentals, including without limitation taxus, juniper, rhododendron, philodendron, ficus, ivy, pothos, lilac, cactus, dizygotheca, euphorbia, fatsia, hedera, coleus, and other varieties, and herbs such as parsley, sage, rosemary, thyme, basil, oregano, garlic, mint, fennel, marjoram, coriander, dill, and the like can also be subjected to the methods of this invention.

Tissues used can come from any desired plant part, including roots, anthers, stems, cotyledons, hypocotyls and flowers. Preferred tissues include meristem explants, whole leaf explants, partial leaf cuttings, leaf punch disks and immature embryos. An especially preferred tissue is a split meristem explant. This latter tissue has been described in the literature by B. Schrammeijer et al., "Meristem Transformation of Sunflower via Agrobacterium," Plant Cell Reports 9: 55-60 (1990).

Agrobacterium Species

Species of Agrobacterium which can be used in plant transformation include Agrobacterium tumefaciens and Agrobacterium rhizogenies. Preferred is an Agrobacterium tumefaciens strain of the nopaline, binary type. Especially preferred is the publicly available Agrobacterium tumefaciens strain EHA101. This strain contains a C58 bacterial chromosome and a disarmed derivative of the Ti plasmid referred to in the literature as TiBO542. [See, e.g., Hood EE, Helmer GL, Fraley RT & Chilton M-D, "The Hypervirulence of Agrobacterium tumefaciens A281 is Encoded in a Region of TiBO542 Outside of T-DNA." J. Bacteriology 168:1291-1301 (1986)].

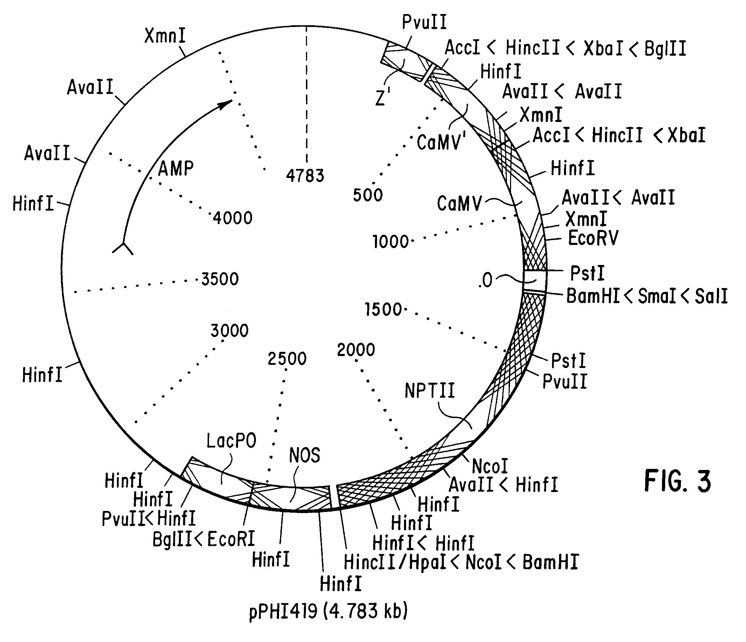

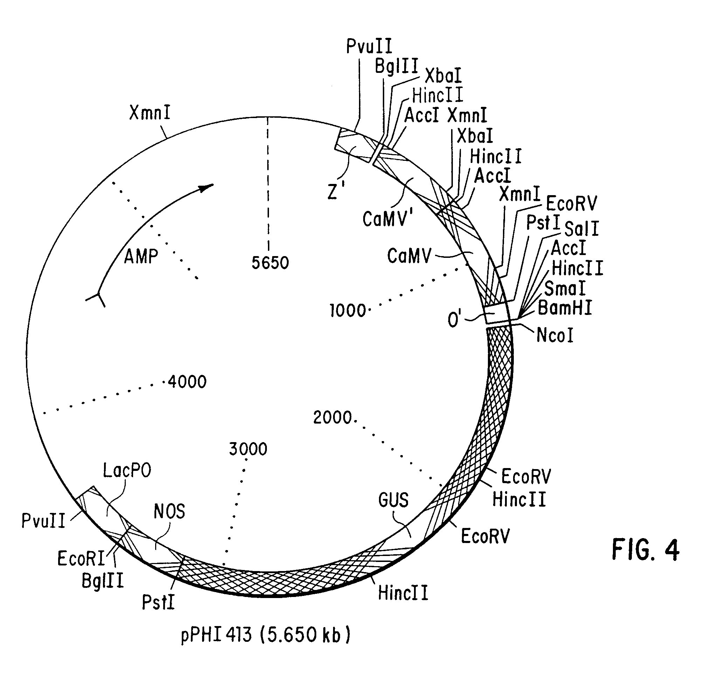

While selection and transformation of Agrobacterium does not per se form a critical part of this invention, in a preferred embodiment strain EHA101 is transformed with plasmids pPHI158 and pPHI167 as shown in Figures 1 and 2, using freeze-thaw transformation. pPHI158 (Figure 1) is constructed by the insertion of linearized, EcoR1 digested plasmid pPHI419 (Figure 3) carrying the plant-expressible marker NPTII near the right border of the 11.6 kb binary pPHI6. pPHI6 also contains the RK2 origin of replication and an ampicillin resistance marker. pPHI167 is constructed in an identical manner except that the linearized EcoR1 fragment of pPHI413 (Figure 4) carrying the GUS gene is inserted into pPHI6. This is referred to in the literature as a binary vector system. [See, e.g., Hoekema A, Hirsch PR, Hooykaas PJJ & Schilperoort RA, "A Binary Plant Vector Strategy Based on Separation of Vir- and T-Regions of the A. tumefaciens Ti Plasmid." Nature 303: 179-180 (1983).]

The bacteria are preferably grown in YEP medium supplemented with 50 $\mu$g/mL kanamycin and 100 $\mu$g/mL carbenicillin to an $OD_{600}$ of 0.5-1.0. For inoculation of plant tissues the bacteria are preferably transferred to inoculation medium. Compositions of various media are as follows:

AB
3 g/L $K_2HPO_4$
1 g/l $NaH_2PO_4$
1 g/l $NH_4Cl$
0.3 g/L $MgSO_4 \cdot 7H_2O$
0.15 g/L KCl
0.01 g/L $CaCl_2$
2.5 mg/l $FeSO_4 \cdot 7H_2O$
YEP
10 g/L Yeast Extract
10 g/L BactoPeptone
5 g/L NaCl
LB
5 g/L yeast extract
10 g/L Bactopeptone
10 g/L NaCl
all above at pH 7.0
Inoculation Buffer
12.5 mM MES at pH 5.7
1 g/L NH4Cl
0.3 g/L MgSO4
Induction Buffer
$\frac{1}{4}$-strength AB medium
3% sucrose
20 mM MES pH 5.5
200 $\mu$M acetosyringone

Example 1

Sunflower transformation

A general method for transformation of sunflower meristem tissues is practiced as follows.

Shelled sunflower seeds are surface sterilized with dilute hypochlorite solution in the usual manner and imbibed overnight (18 hours) in the dark at 26°C on moist filter paper to initiate germination. The following morning, the cotyledons and the emerging root radical are removed and the explant containing the meristem is cultured overnight on medium 374B-GA, which contains Murashige & Skoog minerals, Shepard vitamins, 3% sucrose, 0.8% agar (Phytagar) and the hormones BAP (0.5 mg/L), IAA (0.25 mg/L) and GA (0.1 mg/L) at a pH of 5.6. 24 hours later the primary leaves are removed, exposing the apical meristem. The meristems are arranged in a 2 cm circle in the center of a petri plate containing a stiff water agar to hold the meristems upright for bombardment purposes. The meristems are bombarded twice in a microparticle bombardment apparatus of the general construction described by Sanford et al. in their European Patent Application, Publication Number 331,885, claiming priority of U.S. Patent Application Serial No. 161,807, filed February 29, 1988. Nitric acid-washed tungsten particles having a mean diameter of 1.8$\mu$m suspended in TE buffer are used, and the explants are positioned 10cm below the stopping plate.

Following the bombardments a small droplet of log phase Agrobacteria (containing the desired modified Ti plasmid) in inoculation buffer at a concentration of OD = 2.0 at 600nm is applied to each meristem. The cultures are incubated on 374B-GA for 3 days and then transferred to 374 medium (374B-GA without hormones but containing 250 $\mu$g/mL cefotaxime to inhibit bacterial growth). The meristem plants emerge in a few days and are allowed to develop for about 2 weeks at 26°C with a 16 hour day. At that point plants can be harvested to measure the level of transformation that has occurred. This can be done, for example, 1) based on the number of stained leaf sectors observed using GUS histochemical staining when the GUS gene is used (submerging the tissue overnight in x-gluc), or 2) by observing the green regions under kanamycin selection when the plasmid transferred by the Agrobacterium contains the NPTII gene or under Basta selection using the BAR gene.

Example 2

Tobacco Leaf Transformation

Tobacco leaves were treated in the same general manner as described in Example 1. The culture medium comprised MS minerals and vitamins, 4% sucrose and 1.5% Gelrite at a pH of 5.8. 10 to 14 days post germination, the first true leaves were harvested and cultured adaxial side up for 24 hours on filter paper moistened with medium comprising MS minerals, B-5 vitamins, 4 mg/L pCPA, 3% sucrose and 0.25 M sorbitol at a pH of 5.8. The leaves were dipped for 10 minutes in an Agrobacterium tumefaciens EHA101/pPHI158 or EHA101/pPHI167 suspension after bombardment and then returned to the culture medium, and 3 days later were transferred to 526 medium, comprising MS mineral elements, B-5 vitamins, 0.5 mg/L BAP, 2.0 mg/L NAA, 3% sucrose, and 0.8% Phytagar at a pH of 5.7, supplemented with 100 $\mu$g/Ml kanamycin sulfate and 250 $\mu$g/mL cefotaxime. The NPTII gene was used as a selectable marker and selection was done in a kanamycin-containing medium. Additional treatment groups included three levels of controls: Group II used bombardment followed by the same Agrobacteria as the test group containing same plasmid but without the NPTII gene (i.e., no selectable marker); Group III used the test Agrobacteria containing the test plasmid but without bombardment; and Group IV used standard particle/plasmid bombardment using the NPTII gene (i.e., no Agrobacteria) (Group IV). The combination of particle bombardment, Agrobacterium cultivations and kanamycin selection pressure did not prevent leaves from forming callus and regenerating plants. Transformation was identified by counting colonies surviving on the medium. Results were as follows:

| Treatment group | # of Explants | Avg # of Colonies |
|---|---|---|
| I | 41 | 15.8 |
| II | 68 | 0 |
| III | 64 | Only at cut point |
| IV | 66 | 0.77 |

From these results there appears to be about a 20-fold increase in the frequency of transformation using the method of this invention, compared to standard particle/plasmid methods.

Example III

Sunflower meristems were transformed in the general manner of Example I. Meristem diameter increased from 50 $\mu$m to over 200 $\mu$m in 2 days in culture prior to bombardment. Plants were easily recovered from meristems after treatment. Transformation was evaluated by counting plants with GUS-positive sectors and comparisons were made using treatment groups divided as in groups I, III and IV of Example II. Results, expressed as the percentage of transformants identified among regenerated plants, were as follows:

| Treatment group | % transformants |
|---|---|
| I | 14.4 |
| III | 0 |
| IV | 0.1 |

From these results there appears to be about a 140-fold increase in the frequency of transformation using the method of this invention, compared to standard particle/plasmid methods.

**Example IV**

A more extensive experiment along the lines of Examples 1 and 2 were conducted using Xanthi (tobacco) leaves. Treatment groups were as follows:

| Group | Wounding | Dipped | Bacteria | Plasmid |
|---|---|---|---|---|
| 1 | Particles[1] | No | None | None |
| 2 | Splitting | No | None | None |
| 3 | Particles[1] | Yes | EHA101 | pPHI167 |
| 4 | Particles[1] | Yes | None | pPHI419 |
| 5 | None | Only | EHA101 | pPHI158 |
| 6 | Particles[1] | No | None | pPHI419 |
| 7 | Splitting | Yes | EHA101 | pPHI158 |
| 8 | Particles[1] | Yes | EHA101 | pPHI158 |

[1] **Two bombardments**

The selection medium included kanamycin, so that only cells transformed to contain the NPTII gene (pPHI158 or pPHI419)) were expected to survive. Cells transformed with pPHI167 (GUS gene) could have been identified by blue staining, but would not yield viable colonies in this experiment. Results were as follows:

| Group | # Explants | Mean # Colonies | Colonies/Leaf |
|---|---|---|---|
| 1 | 31 | 0 | 0 |
| 2 | 40 | 0 | 0 |
| 3 | 34 | 0 | 0 |
| 4 | 55 | 0 | 0 |
| 5 | 64 | colony development at excision point only | |
| 6 | 48 | 0.15±0.08 | 0.36 |
| 7 | 37 | 2.5±0.45 | 4.0 |
| 8 | 51 | 14.35±1.9 | 36.5 |

The first five groups were controls: Group 1 to evaluate the effect of bombardment only on colony development; Group 2 to evaluate the effect of wounding using a scalpel cut to split the leaf; Group 3 to evaluate particle wounding as in Group 1 but with functional bacteria that contained GUS only (no selectable marker) to establish a baseline; and Group 4 to evaluate whether the Agrobacteria were needed if microparticle wounding is used. None of these treatments were expected to produce viable colonies on selection medium, and all of these developed 0 colonies on selection medium. Group 5 was a control to confirm the need for tissue wounding, and colonies developed only at the excision point as expected.

Group 6 was a positive control using normal particle gun methods, and Group 7 was a positive control using wounding by a simple scalpel cut to split the leaf. Group 8 used the method of this invention. The method of this invention showed approximately a 100-fold improvement in frequency of transformation compared to a conventional microparticle bombardment method and a 9-fold improvement in comparison to a conventional Agrobacterium transformation method which used a split leaf specimen.

**Example V**

The methods of this invention were compared to the method in which tissues are bombarded with microparticles to which transformed Agrobacteria have been applied, as described in the parallel application EP-A-0 486 234. Results were as follows:

| Bombard | Dia. | Material | Conc. | Meristems | Sectors | % |
|---------|------|----------|-------|-----------|---------|-----|
| w/bact. | ~1.3μm | gold | Dried ( | 121 | 4 | 3.3 |
|         |      |          | in YEP ( | 125 | 3 | 2.4 |
| 2x pre | 1.8μm | tungsten | OD=2 | 106 | 8 | 7.5 |
|        |       |          |      | 138 | 21 | 15.2 |
| 4x pre | 1.8μm | tungsten | OD=2 | 54 | 8 | 14.8 |
| 6x pre | 1.8μm | tungsten | OD=2 | 83 | 9 | 10.8 |
| 2x pre | 1.3μm | tungsten | OD=2 | 73 | 15 | 20.3 |
| 2x pre | 1.8μm | tungsten | OD=2 | 80 | 14 | 17.5 |
| 2x pre | 2.4μm | tungsten | OD=2 | 70 | 8 | 11.4 |
| 2x pre | 1.8μm | tungsten | OD=2 | 39 | 7 | 17.9 |
| 2x pre | 1.8μm | tungsten | OD=4 | 75 | 7 | 9.3 |
| 2x pre | 1.8μm | tungsten | OD=6 | 74 | 7 | 9.5 |

From this it was concluded that although bombarding tissues with Agrobacteria dried onto particles is an effective method of transformation using Agrobacteria, it is not as effective as bombarding first and then applying Agrobacteria as a droplet of suspension to the wound site.

**Claims**

1. A method for transformation of cells of a plant by insertion of genetic material into the genome of the cells, comprising the steps of
   (a) preparing bacteria of an Agrobacterium species, which bacteria have been transformed to include in their T-DNA the genetic material to be inserted into the genome of the cells;
   (b) perforating a tissue from the plant by microprojectile bombardment; and
   (c) treating the perforated tissue with the transformed Agrobacteria;
   whereby the Agrobacteria incorporate the T-DNA including the inserted genetic material into the genome of the cells.

2. A method according to Claim 1 wherein the tissue is a meristem explant.

3. A method according to Claim 1 wherein the tissue is a member selected from the group consisting of whole leaf explants, partial leaf cuttings, and leaf punch disks.

4. A method according to Claim 1 wherein the tissue is immature embryos.

5. A method according to any one of the preceding claims wherein the plant is a monocot selected from the group consisting of maize, sorghum, triticale, barley, oats, rye, wheat, onions and rice.

6. A method according to any one of claims 1 to 4 wherein the plant is a dicot selected from the group consisting of soybean, alfalfa, tobacco, brassicas, sunflower, cucurbits, potatoes, peppers and tomatoes.

7. A method of producing whole, fertile, plants, the cells of which have been transformed by insertion of genetic material into their genome, comprising the steps of
   (a) preparing bacteria of an Agrobacterium species, which bacteria have been transformed to insert in their T-DNA the genetic material to be inserted into the genome of the plant cells;

EP 0 486 233 B1

(b) perforating by microprojectile bombardment a tissue from a plant of the species and genotype to be transformed;

(c) treating the perforated tissue with the transformed Agrobacteria, whereby the Agrobacteria incorporate genetic material comprising the inserted genetic material into the genome of the cells to produce transformed cells; and

(d) regenerating the transformed cells to produce whole plants.

8. A method according to any one of claims 1 to 7, further comprising the step of growing the bombarded, Agrobacterium-treated tissue in a selection medium in which only transformed cells are viable, prior to regeneration.

9. A method according to claim 7 wherein the tissue is as defined in any one of claims 2 to 4.

10. A method according to claim 7 wherein the plant is as defined in claim 5 or 6.

**Patentansprüche**

1. Verfahren zur Transformation von Zellen einer Pflanze durch Insertion von genetischem Material in das Genom von Zellen, umfassend die Stufen

(a) Herstellen von Bakterien einer Agrobacterium-Art, wobei die Bakterien so transformiert wurden, daß sie in ihrer T-DNA das in das Genom der Zellen zu insertierende genetische Material umfassen;

(b) Perforieren eines Gewebes einer Pflanze durch Mikroprojektilbeschuß; und

(c) Behandeln des perforierten Gewebes mit den transformierten Agrobakterien, wobei die Agrobakterien die T-DNA, einschließlich des in das Genom der Zellen insertierten genetischen Materials beherbergen.

2. Verfahren nach Anspruch 1, dadurch **gekennzeichnet,** daß das Gewebe ein Meristemexplantat ist.

3. Verfahren nach Anspruch 1, dadurch **gekennzeichnet,** daß das Gewebe aus der Gruppe, bestehend aus Explantaten ganzer Blätter, Blatteilschnitten und ausgestanzten Blattscheiben, ausgewählt wird.

4. Verfahren nach Anspruch 1, dadurch **gekennzeichnet,** daß das Gewebe unreife Embryonen sind.

5. Verfahren nach einem der vorausgehenden Ansprüche, dadurch **gekennzeichnet,** daß die Pflanze eine monokotyle Pflanze, ausgewählt aus der Gruppe, bestehend aus Mais, Sorghumhirse, Tritikale, Gerste, Hafer, Roggen, Weizen, Zwiebeln und Reis, ist.

6. Verfahren nach einem der Ansprüche 1 bis 4, dadurch **gekennzeichnet**, daß die Pflanze eine dikotyle Pflanze, ausgewählt aus der Gruppe, bestehend aus Sojabohnen, Alfalfa, Tabak, Rüben, Sonnenblumen, Kürbisgewächsen, Kartoffeln, Paprikagewächsen und Tomaten, ist.

7. Verfahren zur Erzeugung ganzer fertiler Pflanzen, deren Zellen durch Insertion von genetischem Material in ihr Genom transformiert wurden, umfassend die Stufen

(a) Herstellen von Bakterien einer Agrobacterium-Art, wobei die Bakterien so transformiert wurden, daß sie in ihrer T-DNA das in das Genom der Pflanzenzellen zu insertierende genetische Material umfassen;

(b) Perforieren eines Gewebes einer Pflanze der zu transformierenden Art und Genotyp durch Mikroprojektilbeschuß; (c) Behandeln des perforierten Gewebes mit den transformierten Agrobakterien, wobei die Agrobakterien das ge

netische Material, einschließlich des in das Genom der Zellen insertierten genetischen Materials beherbergen, wodurch transformierte Zellen gebildet werden; und

(d) Regenerieren der transformierten Zellen unter Erzeugung ganzer Pflanzen.

8. Verfahren nach einem der Ansprüche 1 bis 7, dadurch **gekennzeichnet**, daß man weiterhin das beschossene von Agrobacterium behandelte Gewebe in einem Selektivmedium, in dem nur transformierte Zellen lebensfähig sind, vor der Regeneration züchtet.

9

**9.** Verfahren nach Anspruch 7, dadurch **gekennzeichnet,** daß das Gewebe wie in einem der Ansprüche 2 bis 4 definiert ist.

**10.** Verfahren nach Anspruch 7, dadurch **gekennzeichnet,** daß die Pflanze wie in Anspruch 5 oder 6 definiert ist.

**Revendications**

**1.** Procédé de transformation de cellules d'un végétal par insertion de matériel génétique dans le génome des cellules, comprenant les étapes consistant à :

(a) préparer des bactéries appartenant à une espèce d'Agrobacterium, lesquelles bactéries ont été transformées pour inclure dans leur ADNt le matériel génétique devant être inséré dans le génome des cellules;

(b) perforer un tissu provenant du végétal par bombardement de microprojectiles ; et

(c) traiter le tissu perforé avec les Agrobacteria transformées; ce par quoi les Agrobacteria incorporent dans le génome des cellules l'ADNt comprenant le matériel génétique inséré.

**2.** Procédé selon la revendication 1, dans lequel le tissu est un explant de méristème.

**3.** Procédé selon la revendication 1, dans lequel le tissu est un élément choisi dans le groupe constitué par les explants de feuille entière, les découpes partielles de feuilles, et les disques découpés à partir de feuilles.

**4.** Procédé selon la revendication 1, dans lequel le tissu est une plantule immature.

**5.** Procédé selon l'une quelconque des revendications précédentes, dans lequel la plante est une monocotylédone choisie dans le groupe constitué par le maïs, le sorgho, la triticale, l'orge, l'avoine, le seigle, le blé, l'oignon et le riz.

**6.** Procédé selon l'une quelconque des revendications 1 à 4, dans lequel la plante est une dicotylédone choisie dans le groupe constitué par le soja, la luzerne, le tabac, les plantes appartenant au genre Brassica, le tournesol, les cucurbitacées, les pommes de terre, les poivrons et les tomates.

**7.** Procédé de production de végétaux entiers fertiles, dont les cellules ont été transformées par insertion de matériel génétique dans leur génome, comprenant les étapes consistant à :

(a) préparer des bactéries appartenant à une espèce d'Agrobacterium, lesquelles bactéries ont été transformées pour insérer dans leur ADNt le matériel génétique devant être inséré dans le génome des cellules des végétaux;

(b) perforer par bombardement de microprojectiles un tissu provenant d'un végétal de l'espèce et du génotype devant être transformé;

(c) traiter le tissu perforé avec les Agrobacteria transformées, ce par quoi les Agrobacteria incorporent du matériel génétique comprenant le matériel génétique inséré dans le génome des cellules pour produire des cellules transformées ; et

(d) régénérer les cellules transformées pour produire des végétaux entiers.

**8.** Procédé selon l'une quelconque des revendications 1 à 7, comprenant en outre l'étape consistant à faire croître, avant régénération, le tissu bombardé traité par Agrobacterium dans un milieu de sélection dans lequel seules les cellules transformées sont viables.

**9.** Procédé selon la revendication 7, dans lequel le tissu est tel que défini dans l'une quelconque des revendications 2 à 4.

**10.** Procédé selon la revendication 7, dans lequel le végétal est tel que défini dans la revendication 5 ou 6.

pPHI 158 (15.856 kb)

FIG. 1

pPHI 167 (16.75 kb)

FIG. 2

FIG. 3

PvuII
AccI < HincII < XbaI < BglII
HinfI
AvaII < AvaII
XmnI
AccI < HincII < XbaI
HinfI
AvaII < AvaII
XmnI
EcoRV
PstI
BamHI < SmaI < SalI
PstI
PvuII

Z'
CaMV'
CaMV
0
500
1000
1500
2000
2500
3000
3500
4000
4783

XmnI
AvaII
AvaII
HinfI
HinfI

AMP
NOS
LacPO
NPTII

HinfI
HinfI
PvuII < HinfI
BglII < EcoRI
HinfI
HinfI
HincII / HpaI < NcoI < BamHI
HinfI < HinfI
HinfI
HinfI
AvaII < HinfI
NcoI

pPHI419 (4.783 kb)

FIG. 4

pPHI 413 (5.650 kb)

14